# EUROPEAN PATENT SPECIFICATION

(11) **EP 1 619 182 B1**
(45) Date of publication and mention of the grant of the patent: **23.07.2014**
(21) Application number: 04722121.3
(22) Date of filing: 19.03.2004
(51) Int. Cl.: C07D 207/26, A61K 31/4015, A61P 25/28, A61P 25/24

(54) **SUBSTANCE EXHIBITING ANTIDEPRESSANT PROPERTY**
SUBSTANZ, DIE ANTIDEPRESSIVE EIGENSCHAFTEN ZEIGT
SUBSTANCE MANIFESTANT UNE PROPRIETE ANTIDEPRESSIVE

(30) Priority: 10.04.2003 RU 2003110288
(43) Date of publication of application: 25.01.2006
(73) Proprietor: Akhapkina, Valentina Ivanovna, 105264 Moscow (RU)
(72) Inventor: AKHAPKINA, Valentina Ivanovna, 105264 Moscow (RU); AKHAPKIN, Roman Vitalievich, 105264 Moscow (RU); ALEKSANDROVSKY, Yury Anatolievich, 125493 Moscow (RU); AVEDISOVA, Alla Sergeevna, 109088 Moscow (RU); VORONINA, Tatyana Aleksandrovna, 119048 Moscow (RU); NESTERUK, Vladimir Viktorovich, 125171 Moscow (RU)
(74) Representative: Dannenberger, Oliver Andre
(86) International application number: PCT/RU2004/000106
(87) International publication number: WO 2004/089893

(56) References cited:
- WO-A2-01/62726
- GB-A- 1 472 154
- RU-C1- 2 050 851
- SU-A1- 797 219
- US-A- 3 956 314
- US-A- 4 145 347
- DATABASE CA [Online] CHEMICAL ABSTRACTS SERVICE, COLUMBUS, OHIO, US; 1983, BOBKOV, YU. G. ET AL: "Pharmacological characteristics of 4-phenylpiracetam, a new phenyl analog of piracetam" XP002558204 retrieved from STN Database accession no. 1983:209935 & BOBKOV, YU. G. ET AL: "Pharmacological characteristics of 4-phenylpiracetam, a new phenyl analog of piracetam" BYULLETEN EKSPERIMENTAL'NOI BIOLOGII I MEDITSINY, vol. 95, no. 4, 1983, pages 50-53, ISSN: 0365-9615
- SPEKTOR S.S. ET AL.: 'Experimentalnaya farmakokinetika karfedona' KHIMIKO-FARMATSEVTICHESKY ZHURNAL vol. 30, 1996, MOSCOW, FOLIUM, pages 3 - 4, XP008040747
- KIM S. ET AL.: 'Determination of carphedon in human urine by solid-phase microextraction using capillary gas chromatography with nitrogen-phosphorus detection' ANALYST vol. 124, no. 11, November 1999, pages 1559 - 1562, XP002904726

## Description

### Field of the Invention

This invention relates to medicine, more particularly to pharmacology, and specifically to antidepressants selectively improving depressed mood with dominating asthenic emotions manifested as melancholy, apathy, volition diminution, anxiety, fears, especially in cases when changed mood is relatively fixed and stable within not only days or weeks, but even months and years, and has effects on behavior, thinking, activity and physiological functions of the organism.

### Description of the Prior Art

The standard antidepressant, which is widely used for treatment of depressed moods, is amitriptyline (5-(3-dimethylaminopropylidene)-10, 11-dihydrodibenzocycloheptine hydrochloride (Mashkovsky M.D., Medications // Pharmacotherapy Aid for Physicians, P. 1, Moscow, "Medicina" Publishers, 1988, p. 90-96).

Amitriptyline, as a standard medication, has the following disadvantages: presence of sedative and clearly manifested anticholinergic effects, relatively slow therapeutic effect, as well as direct dose-dependence effect requiring to continuously increase a dose, if applied in a course treatment, gradually reduce the dose after withdrawal of the medication, limitations in professions requiring attention and quick decision-making.

### Summary of the Invention

The objective of this invention is to develop a medication having a high antidepressant activity combined with the absence of the adverse reactions peculiar to amitriptyline.

In compliance with the said objective it is possible to obtain technical results causing an increased antidepressant effect after one dose, absence of sedative and cholinergic effects, avoidance of tolerance and the withdrawal syndrome at course treatment, removal of limitations for professional activities.

The said technical results can be achieved due to the fact that N-carbamoyl-methyl-4-phenyl-2-pyrrolidone (phenotropyl) is used as the antidepressant agent.

Phenotropyl is known as a substance having hypotensive activity (SU Nº 797219, A 61 K 21/40); a substance having nootropic activity (RU Nº 2050851, A 61 K 31/40); an antiischemic agent (RU Nº 2183117, A 61 K 31/405).

WO-A-01/62726 relates to 2-oxo-1-pyrrolidine derivatives. It discloses the compound phenotropyl as compound number 2 on page 67. In the document it is stated that compounds of formula I may be used for the treatment of neurological disorders, including depression. A group of compounds, including phenotropyl, has been excluded from the claims of WO-A-01/62726 by way of a proviso. WO-A-01/62726 does not contain experimental data with regard to phenotropyl.

US-A-3956314 discloses phenyl-2-pyrrolidones as anxiolytic and antidepressant agents. These compounds are structurally distinct from phenotropyl.

Antidepressant properties of phenotropyl are unknown. Phenotropyl has not been used as a medication used for treatment of depressions.

### Detailed Description of the Preferred Embodiment

The study of the phenotropyl antidepressant activity has been carried out in accordance with the Guidebook for Experimental (Preclinical) Studies of New Pharmaceuticals of the RF Ministry of Public Health (Andreeva N.I., the Methodical Guidelines for Studying Antidepressant Activity of Pharmaceuticals, ZAO IIA "Remedium", 2000, p. 121-125).

The study results have been processed statistically. The average values and their standard deviations for each group have been calculated. The reliability of the differences between the experimental and the control groups has been checked by a dispersion analysis and the Student's method (Borovikov V.P., Statistics: the art of computerized analysis of data. For professionals // Saint-Petersburg - Piter - 2001. 247 p.).

### Example 1. A study of the antidepressant action of phenotropyl with the use of the Porsolt method of behavioral despair (helplessness).

The Porsolt method of behavioral despair (helplessness) (Porsolt R.D., Anton G., Blavet N. et al. Behavioral despair in rats: a new model sensitive to antidepressant treatment // Europ. J. Pharmacol. 1978-v.47.-p. 379-391) is the basic model for assessing antidepressants (Andreeva N.I., the Methodical Guidelines for Studying Antidepressant Activity of Pharmaceuticals, // Guidebook for Experimental (Preclinical) Studies of New Pharmaceuticals of the RF Ministry of Public Health.- ZAO IIA "Remedium".- 2000.- p. 121-125). The studies have been conducted on white outbred male rats having the weight of 230-250 grams. The animals have been put into a water reservoir having 40 cm in diameter and the depth of 60 cm, for the rats cannot either run out of the reservoir or find a support in it. The water temperature has been maintained at the level of 25 °C, the experiments have been conducted within the period from 12 A.M. to 4 P.M. After coming into water the animals begin displaying energetic motion activity aimed at finding a way out of the aversive (troublesome) situation, but then they stop their attempts and take a typical position in the water, while being fully immovable or making insignificant motions necessary for keeping their heads over the water surface. The depressive condition intensity rate in this test is the duration of immobility. The immobility condition has been assessed visually, determining its duration within 10 minutes of the observations. Substances having antidepressant activity alleviate this condition by reducing the immobility time.

The studies have been conducted using animals divided into groups. The animals from the experimental groups have received single doses of phenotropyl in the amounts of 25, 50 and 100 mg/kg or the standard amitriptyline medication in the amount of 10 mg/kg (ampoule solution made by Spofa, the Czech Republic) in the volume corresponding to the dose. The animals from the control group have received distilled water in the same dosage. All the substances have been given intragastrically 40 minutes before the an experiment.

It has been established that during the behavioral despair test the control group animals, after being put into a water reservoir and making active attempts to come out of the aversive situation, have revolutioned into the immobility state reflecting their depressive condition. Phenotropyl in single doses of 50 and 100 mg/kg has a pronounced antidepressant effect during this test (see Table 1). The medication effect coming after doses of 50 and 100 mg/kg has been displayed as statistically reliable reduction (by 1.6 times) in the time of immobility of the rats, as compared to the control group. Amitriptyline, as the standard antidepressant, has also reliably reduced the immobility time.

Thus, judging by its antidepressant effect in the behavioral helplessness test, phenotropyl has activity similar to that of amitriptyline.

### Example 2. The study of the phenotropyl antidepressant action with the use of the method of involuntary swimming with freely moving wheels according to Nomura.

The method of involuntary swimming in a reservoir with freely moving wheels (Nomura S., Shimizu J., Kinjo M. et al. A new behavioral test for antidepressant drugs// Eur. J. Pharmacol.-1982.-v.83.-N 3-4.-p. 171-175) is widely used for assessing the action of antidepressant agents. The installation is a reservoir having dimensions of 64x30x42 cm and divided into four equal sections where wheels arc located. The reservoir is filled with water at the temperature of 25 °C, coming to the medium line of the wheels. The studies have been conducted with white outbred male rats having the weight from 230 to 250 grams. The rats have been put into each section in the position where their heads are opposite to the wheels, and then the wheel speed has been recorded for 10 minutes with the use of electromechanical counters. The number of wheel revolutions made by the rat for 10 minutes is recorded.

This study has been conducted with animals from different groups. The rats from the experimental groups have received single doses of phenotropyl in the amounts of 25, 50 and 100 mg/kg or the standard amitriptyline medication in the amount of 10 mg/kg (ampoule solution made by Spofa, the Czech Republic) in the volume corresponding to the dosage. The animals from the control group have received distilled water in the same dosage. All the substances have been given intragastrically 40 minutes before the an experiment.

It has been established that during the test of involuntary swimming in the reservoir with wheels the rats from the control group first try to go out of the water, but since the wheels are moving freely, their attempts are not successful, and the animals soon give up, "hovering" in the water. The number of wheel revolutions for 10 minutes of observation is 58,8 ± 14,3 (see Table 2). After a single dose of phenotropyl given to the rats, their manifestations of depressive conditions have been reduced, which has been evidenced by the fact that the animals begin revolutioning the wheels actively. Thus, after introducing a phenotropyl dose of 100 mg/kg, the rats have made 121,8 ± 23,7 revolutions for 10 minutes of observations. The evidence of the antidepressant effect of phenotropyl in doses of 25, 50 and 100 mg/kg have been statistically reliable, as compared to the data obtained for the control group. Amitriptyline, similarly to phenotropyl, also increases the rat activity, reliably increasing the number of wheel revolutions.

Thus, judging by the results of the involuntary swimming test, phenotropyl has pronounced antidepressant action and is not inferior to amitriptyline as to its activity.

### Example 3. The study of the phenotropyl antidepressant activity with the use of the reserpine depression method.

The method of reserpine depression according to Bernardi (Bernardi D., Paglialunga S., Jori A. Peripheral and central components in the hyperthermic effect of desipramine in rezerpinized rats // J. Pharm. Pharmacol., 1968, vol. 20, p. 204-209) is one of the basic models for assessing antidepressants in studies of new pharmaceuticals. The studies have been conducted with white outbred male rats having the weight from 200 to 240 grams. Reserpine has been introduced intraperitoneally, as a single dose of 2.5 mg/kg 2 hours before the introduction of phenotropyl (intraperitoneally, single doses of 25, 50, 100 and 200 mg/kg) and 3 hours before starting the study. The animals from the control group have received intraperitoneally distilled water only in the same volumes as the animals from the experimental groups. The antidepressant activity of phenotropyl has been assessed by its effect on sedation (suppression of the orientation and motion activity), intensity of ptosis and diarrhea, which have been caused by reserpine in the rats.

The orientation and motion activity of the animals has been studied for three minutes in a special activity meter (Varimex). The intensity of ptosis and diarrhea has been assessed on the standard 4-point scale (Mashkovsky M. D., Andreeva N.I., Polezhaeva A.I. Pharmacology of Antidepressants. Moscow, Medicine Publishers, 1983, p. 194-199).

The study results, as presented in Table 3, show that a single dose of phenotropyl prominently antagonizes the reserpine syndrome: the intensity of ptosis is reliably reduced by 1.4 and 1.8 times, respectively, after introducing doses of 50, 100 and 200 mg/kg; the diarrhea intensity is reliably reduced by 1.7 times after a dose of 50 mg/kg, by 2.4 times after a dose of 100 mg/kg, and by 2.2 times after a dose of 200 mg/kg; the orientation and motion activity of the animals is increased at the background of phenotropyl doses of 25 and 50 mg/kg and reliably corresponds to the control level when administering doses of 100 and 200 mg/kg.

Thus, judging by the results of the reserpine depression test, phenotropyl has pronounced antidepressant activity and displays the motion activating component.

### Example 4. The study of the phenotropyl antidepressant activity by the haloperidol catalepsy method.

As one of the indicators for antidepressant antagonism in relation to depriming effects of neuroleptics, neuroleptic-induced catalepsy is used. The maintenance of catalepsy in animals is assessed by their ability to maintain set or unusual poses for a definite time (Morpurgo C. Effects of antiparkinson drugs on phenothiazine induced catatonia reaction // Arch. Int Pharmacodyn., 1962, vol. 137 Nº 1-2, p. 84-94). Reduction or prevention of catalepsy is one of the factors showing antidepressant activity in studied substances.

The studies have been conducted with white outbred male mice having the weight from 18 to 24 grams. Haloperidol is introduced intraperitoneally in a single dose of 0.5 mg/kg. Phenotropyl has been introduced intraperitoneally in doses of 50, 100 and 200 mg/kg in 15 minutes after the introduction of haloperidol. The catalepsy intensity has been assessed by time (in seconds) of keeping the animals in an uncomfortable pose where their front legs have been put onto a bar having the height of 4 cm, in 60, 120 and 180 minutes after introducing the said medications. It is shown in Table 4 that phenotropyl clearly antagonizes haloperidol-induced catalepsy. Phenotropyl in doses of 50 and 100 mg/kg fully prevents the development of catalepsy in 60 minutes and reduces its development level 3.2 and 14 times, respectively, in 120 minutes after the introduction of the medication, and 13.2 times with a dose of 50 mg/kg in 180 minutes, fully stopping its development in 180 minutes with a dose of 100 or 200 mg/kg.

The study results show that, according to the haloperidol catalepsy test, phenotropyl has antidepressant activity.

### Example 5. The study of the phenotropyl antidepressant activity under the Hamilton rating scale during clinical studies.

The Hamilton rating scale for primary depressive illness (Hamilton M. Development of a rating scale for primary depressive illness // Br J Soc Clin Psychol. 1967 Dec;6(4):278-96) is one of the most widely used scales for assessing depression in the clinical practice.

The material for clinical studies has comprised two homogenous groups of patients having depressive illnesses (40 persons) aged 20 - 42, out of which 20 patients have taken amitriptyline in the daily dose of 75 mg (25 mg three times a day), and 20 patients have taken phenotropyl in the daily dose of 100 mg as a single dose in the morning time. For a comparative study of the medications antidepressant activity the patients have been enlisted for the study according to the following criteria: the informed consent is available, a light or moderate depressive episode (F32.0 and F32.1 according to ICD-10) is present, the total score under the Hamilton rating scale is not lower than 18, no contraindications to taking amitriptyline and phenotropyl. The patients have been relatively uniformly assigned to the said groups according to meaningful factors, such as sex and age, time for which the condition exists and syndrome characteristics. The condition existence time varied from 1 to 2 years. The therapy efficiency has been assessed on the basis of a degree of reduction of the total score under the Hamilton scale, namely: full effect - reduction by more than 50%; partial reduction - from 25 to 50%; no effect - reduction by less than 25%. The condition of the patients has been assessed during the background studies and at each 7th day of the therapy for the whole 6 weeks of the therapy. In total, seven examinations have been carried out. The comparative data on the general efficiency pf the medications, as given in Table 5, evidence a rather quick reduction of the depressive illness psychopathological symptoms in the process of administering phenotropyl and amitriptyline. However, the antidepressant effect of phenotropyl has been more pronounced on the 7th and 14th days of the therapy. The full reduction has been attained in 25% and 40% of the patients, respectively. The full reduction is attained in 70% and 80% of the patients on the 21st and 28th days of the therapy, i.e., two weeks earlier than for the amitriptyline therapy. From the 28th day of the therapy the number of the patients experiencing the maximum effect has not been changed either on the 35th or the 42nd days of the therapy. Out of 20% of the patients experiencing a partial reduction of the depressive symptoms, 15% have been patients with the reduction from 25% to 50%, and 5% - patients with the reduction by less than 25%. At the background of taking phenotropyl no cases of sedative and cholinergic effects have been recorded, the workability has been improved, asthenia and inhibition have been corrected.

The clinical study results show that phenotropyl has pronounced antidepressant activity according to the Hamilton rating scale of depressive illness.

The conducted studies evidence the fact that phenotropyl has pronounced antidepressant activity in the conditions required for the reserpine depression method and the haloperidol catalepsy method, in the conditions required for the behavioral despair method and the involuntary swimming method, as well as in the conditions of a clinical study under the Hamilton rating scale of depressive illness. The medication effect has been observed for doses of 25, 50, 100 and 200 mg/kg during the experimental studies in animals. The clinical studies of phenotropyl in the dose of 100 mg show its high antidepressant activity in light depressive conditions and depressions of moderate degree. Also, the advantage of phenotropyl is that it may be taken once a day. The course administration of this medication does not induce tolerance and the withdrawal syndrome. As to its antidepressant activity, phenotropyl is not inferior to the standard amitriptyline medication. Phenotropyl is of interest for psychiatric and neurological practice as well as for the emergency medicine and the accident medicine.

### Industrial Applicability

This invention is industrially applicable and may be most successfully used as antidepressants selectively improving the condition of a depressive mood with dominating asthenic emotions manifested as melancholy, apathy, volition diminution, anxiety, fears, especially in cases when changed mood is relatively fixed and stable within not only days or weeks, but even months and years, and has effects on behavior, thinking, activity and physiological functions of the organism. The inventive substance does not require any new technologies or equipment.

**Table 1.**

| The effect of phenotropyl after single introduction on the time of immobility in the conditions of the behavioral despair method according to Porsolt | | |
|---|---|---|
| Substances | Dose, mg/kg | Time of immobility, seconds |
| Control | - | 342.3 ± 58.1 |
| Phenotropyl | 25 | 275.5 ± 84.2 |
| Phenotropyl | 50 | 185.7 ± 49.5* |
| Phenotropyl | 100 | 158.4 ± 43.2* |
| Amitriptyline | 10 | 166.1 ± 46.5* |

| | | |
|---|---|---|
| * - Difference with the control group is valid at P < 0.05. | | |

**Table 2.**

| Antidepressant action of phenotropyl during the involuntary swimming test on rats put into a reservoir with wheels, according to Nomura | | |
|---|---|---|
| Substance | Dose, mg/kg | Number of wheel revolutions for 10 minutes |
| Control | | 58.8 ± 14.3 |
| Phenotropyl | 25 | 89.3 ± 17.2* |
| Phenotropyl | 50 | 102.7 ± 16.1* |
| Phenotropyl | 100 | 121.8 ± 23.7* |
| Amitriptyline | 10 | 115.1 ± 24.6* |

| | | |
|---|---|---|
| * - Difference with the control group is valid at P < 0,05. | | |

**Table 3.**

| Effect of single introduction of phenotropyl on reserpine depression. | | | | |
|---|---|---|---|---|
| Substance | Dose, mg/kg | Ptosis, points | Diarrhea, points | Number of pulses in activity meter |
| Control | - | 0.6±0.2 | 0 | 33.6±2.1 |
| Reserpine | 2.5 | 3.6±0.2 | 2.4±0.1 | 11.8±2.2 |
| Reserpine + Phenotropyl | 2.5 | 3.0±0.2 | 2.0±0.1 | 20.3±1.4 |
| | 25 | | | |
| Reserpine + Phenotropyl | 2.5 | 2.5±0.2* | 1.4±0.2* | 22.8±1.2* |
| | 50 | | | |
| Reserpine + Phenotropyl | 2.5 | 2.0±0.2* | 1.0±0.2* | 30.6±2.4* |
| | 100 | | | |
| Reserpine + Phenotropyl | 2,5 | 2,0±0,1* | 1,1±0,2* | 33.9±3.2* |
| | 200 | | | |

| | | | | |
|---|---|---|---|---|
| * - Difference with the control group is valid at P < 0,05. | | | | |

**Table 4.**

| Effect of single introduction of phenotropyl on haloperidol catalepsy. | | | | |
|---|---|---|---|---|
| Substance | Dose, mg/kg | Catalepsy (in seconds) | | |
| | | 60 minutes | 120 minutes | 180 minutes |
| Haloperidol | 0.5 | 1.3±0.8 | 27.5±8.5 | 63.4±2.8 |
| Haloperidol + Phenotropyl | 0.5 | 0.0±0.0* | 8.2±3.2* | 4.5±2.5* |
| | 50 | | | |
| Haloperidol + Phenotropyl | 0.5 | 0.0±0.0* | 2.0±1.0* | 0.0±0.0* |
| | 100 | | | |
| Haloperidol + Phenotropyl | 0.5 | 0.0±0.0* | 11.8±2.1* | 0.0±0.0* |
| | 200 | | | |

| | | | | |
|---|---|---|---|---|
| * - Difference with the haloperidol group is valid at P < 0,05. | | | | |

**Table 5.**

| Phenotropyl antidepressant activity according to the Hamilton rating scale for depressive illness. | | | | | | | | | | | | | | | | | | | |
|---|---|---|---|---|---|---|---|---|---|---|---|---|---|---|---|---|---|---|---|
| Medications (dose in mg) | | Therapy day | | | | | | | | | | | | | | | | | |
| | | 7th | | | 14th | | | 21st | | | 28th | | | 35th | | | 42nd | | |
| | | ≥ 50 | 25 - 49 | < 25 | ≥ 50 | 25 - 49 | < 25 | ≥ 50 | 25 - 49 | < 25 | > 50 | 25 - 49 | < 25 | ≥ 50 | 25 - 49 | < 25 | ≥ 50 | 25 - 49 | < 25 |
| Amitriptyline | n | 0 | 4 | 16 | 4 | 6 | 10 | 8 | 6 | 6 | 14 | 2 | 4 | 17 | 1 | 2 | 17 | 1 | 2 |
| 75 mg | % | 0 | 20 | 80 | 20 | 30 | 50 | 40 | 30 | 30 | 70 | 10 | 20 | 85 | 5 | 10 | 85 | 5 | 10 |
| Phenotropyl | n | 0 | 5 | 15 | 8 | 6 | 6 | 14 | 2 | 4 | 16 | 3 | 1 | 16 | 3 | 1 | 16 | 3 | 1 |
| 100 mg | % | 0 | 25 | 75 | 40 | 30 | 30 | 70 | 10 | 20 | 80 | 15 | 5 | 80 | 15 | 5 | 80 | 15 | 5 |

## Claims

1. N-carbamoyl-methyl-4-phenyl-2-pyrrolidone for use in the treatment of depression.

## Patentansprüche

1. N-Carbamoylmethyl-4-phenyl-2-pyrrolidon zur Verwendung bei der Behandlung von Depression.

## Revendications

1. N-Carbamoyl-méthyl-4-phényl-2-pyrrolidone pour une utilisation dans le traitement de la dépression.
